# EUROPEAN PATENT SPECIFICATION

(11) **EP 1 153 598 B1**
(45) Date of publication and mention of the grant of the patent: **01.03.2006**
(21) Application number: 01110126.8
(22) Date of filing: 03.05.2001
(51) Int. Cl.: A61Q 5/06, A61Q 5/08, A61K 8/49, A61K 8/35

(54) **Hair dye composition comprising hydrogen peroxide and two specific direct dyes**
Haarfärbemittel enthaltend Wasserstoffperoxid und zwei spezifische direktziehende Farbstoffe
Composition de coloration capillaire comprenant du peroxyde d'hydrogène et deux colorants directs spécifiques

(30) Priority: 11.05.2000 JP 2000138467
(43) Date of publication of application: 14.11.2001
(73) Proprietor: KAO CORPORATION, Tokyo 103-8210 (JP)
(72) Inventor: Matsunaga, Kenichi, Kao Corporation, Sumida-ku, Tokyo 131-8501 (JP); Miyabe, Hajime, Kao Corporation, Sumida-ku, Tokyo 131-8501 (JP)
(74) Representative: HOFFMANN EITLE

(56) References cited:
- EP-A- 0 827 737
- EP-A- 1 133 977
- WO-A-97/20545
- DE-A- 3 814 685
- US-A- 5 688 291

## Description

### Technical Field

The present invention relates to a hair dye capable of dyeing the hair in a natural, deep, brown to black color tone while bleaching it.

### Background Art

In Japanese Language Laid-Open Publication (PCT) No. Hei 8-501322, disclosed is a cationic direct dye capable of dyeing the hair in a deep color and having excellent color fastness. In Japanese Language Laid-Open Publication (PCT) No. Hei 10-502946, proposed is a hair dye which comprises the direct dye disclosed in the above Japanese Language Laid-Open Publication (PCT) No. Hei 8-501322 and an oxidizing agent, and dyes the hair under basic conditions while bleaching the hair.

Among the direct dyes described in Japanese Language Laid-Open Publication (PCT) No. Hei 8-501322, however, only those having a color tone ranging from yellow to red are exemplified specifically as the cationic direct dyes in Japanese Language Laid-Open Publication (PCT) No. Hei 10-502946. Blue dyes indispensable for dyeing the hair in brown or black are omitted. The present inventors conducted an investigation on its reason and found that the blue direct dyes represented by the formula (1) (X = S or O) or the formula (6) in Japanese Language Laid-Open Publication (PCT) No. Hei 8-501322 lose their color immediately owing to the reactivity with hydrogen peroxide under basic conditions. In accordance with the technique disclosed in Japanese Language Laid-Open Publication (PCT) No. Hei 10-502946, it was therefore impossible to dye the hair in a natural brown or black color.

EP 1 133 977, which represents prior art under Art. 54(3), (4) EPC, shows a hair dye composition comprising in a first part Basic Blue 99 and a compound of the formula 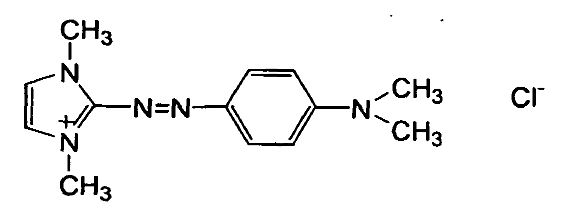 and in a second part hydrogen peroxide.

US 5688291 reveals a composition for simultaneously lightening and colouring hair which comprises a category-2 bleach comprising a persulphate salt and an oxidatively stable disperse dye selected from azo and anthraquinone dyes. The dyestuffs Disperse Blue 3, 7 and 72 are cited as appropriate dyestuffs.

EP 0 827 737 describes an agent for lightening and improving the gloss of human hairs which includes a hydrogen peroxide and at least a direct hair dye. Disperse Violet 4 and Basic Blue 99 are used.

A 2-component preparation consisting of a first powdery component, including at least one dye and an alkali agent, and a second liquid component consisting of an aqueous hydrogen peroxide solution is disclosed in DE 3814685: Basic Blue 99 and Disperse Blue I are cited as appropriate dyes.

### Disclosure of the Invention

An object of the present invention is therefore to provide a hair dye capable of dyeing the hair in a natural, deep, brown to black color tone while bleaching the hair.

The present inventors have found that a hair dye which can satisfy the above-described requirement is available by adding, to a hair bleaching composition, a combination of the cationic direct dye described in Japanese Language Laid-Open Publication (PCT) No. Hei 8-501322 and having a yellow to red color tone, and a specific cationic direct dye having a blue to purple color tone.

In the present invention, there is thus provided a hair dye composition having a pH ranging from 8 to 12 and comprising a first component part containing an alkali agent and a direct dye, and a second component part containing hydrogen peroxide, wherein the first component part contains, as the direct dye, (A) a first direct dye represented by any one of the following formulas (1) to (8): 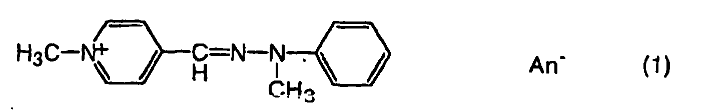 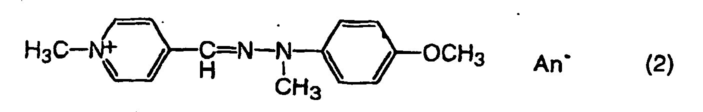 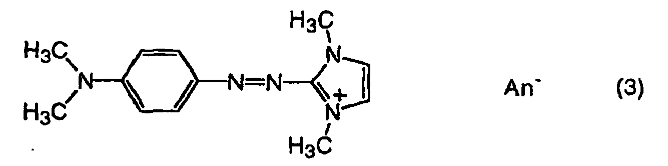 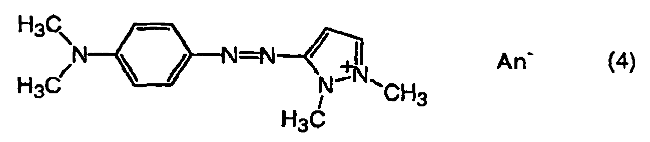 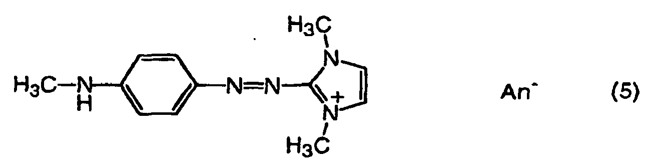 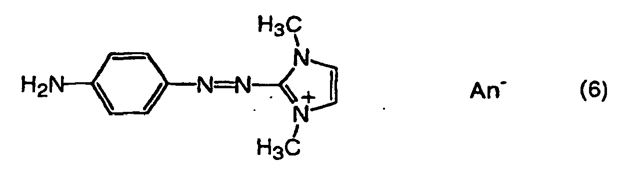 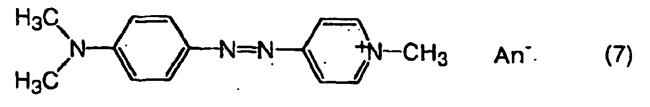 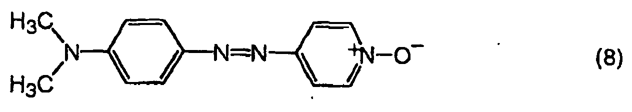 [wherein, An⁻ represents an anion]; and (B) a second direct dye selected from Basic Blue 47, Disperse Violet 1, Disperse Violet 4, Disperse Violet 15 and Disperse Violet 27.

In the present invention, there is also provided a hair dye having a pH ranging from 8 to 12 and comprising a first component part containing an alkali agent, a second component part containing hydrogen peroxide and a third component part containing a direct dye, wherein the third component part contains, as the direct dye, (A) the above-described first direct dye and (B) the above-described second direct dye.

### Best Modes for Carrying Out the Invention

The hair dye of the present invention is either one of a two-part hair dye composed of a first component part containing an alkali agent and a direct dye and a second component part containing hydrogen peroxide; or a three-part hair dye composed of a first component part containing an alkali agent, a second component part containing hydrogen peroxide and a third component part containing a direct dye. The first direct dye as the component (A) and the second direct dye as the component (B) are incorporated in the first component part in the case of a two-part hair dye, while they are incorporated in the third component part in a three-part hair dye.

In the first direct dye to be used as component (A), examples of the anion represented by An⁻ in the formulas (1) to (7) include chloride, bromide, iodide, trichlorozincic acid, tetrachlorozincic acid, sulfuric acid, hydrosulfuric acid, methyl sulfate, phosphoric acid, formic acid and acetic acid ions.

The first direct dyes (1) to (8) can be used either singly or in combination and its (or their) content is preferably 0.001 to 10 wt.%, especially 0.01 to 5 wt.%, each based on the whole composition [mixture of the first and second component parts (or, further third component part); which will equally apply hereinafter).

The second direct dyes to be used as component (B) may be incorporated either singly or in combination and its (or their) content is preferably 0.001 to 10 wt.%, especially 0.01 to 5 wt.%, each based on the whole composition.

Examples of the alkali agent to be incorporated in the fist component part include alkanolamines such as monoethanolamine and isopropanolamine, guanidium salts such as guanidine carbonate and ammonia. These alkali agents may be used either singly or in combination. Its (their) content preferably ranges from 0.1 to 10 wt.%, more preferably 0.5 to 5 wt.%, especially 1 to 3 wt.% based on the whole composition from the viewpoints of sufficient hair dyeing effects and reduced irritation to the scalp. The pH of the first component part is preferably adjusted to 8 to 12, especially 8.5 to 11.

The content of hydrogen peroxide in the second component part preferably falls within the range of 1 to 12 wt.% based on the whole composition from the viewpoints of sufficient bleaching hair dyeing effects and reduced irritation to the scalp.

In addition to the above-described components, water and those ordinarily used in the field of cosmetics can be incorporated in the hair dye of the present invention. Examples of such an optional component include natural or synthetic polymer compounds, oils and fats, hydrocarbons, higher alcohols, monohydric or polyhydric alcohols, silicone derivatives, amine oxides, amino acid derivatives, protein derivatives, antiseptics, sequestering agents, antioxidants, stabilizers for hydrogen peroxide, plant extracts, vitamins, pigments, ultraviolet absorbers, perfumes and pH regulators.

The hair dye is used by mixing the first and second component parts in the case of a two-part hair dye, or mixing the first, second and third component parts in the case of a three-part hair dye upon usage and applying the mixture to the hair. It is preferred that in the former case, the mixing ratio of the first component part to the second component part is set at 2:1 to 1:3 (weight ratio), while in the latter case, the mixing ratio of the first component part to the second component part is set at 2:1 to 1:3 (weight ratio) and the third component part is added in an amount of 1/20 to 1 (weight ratio) relative to the first component part. These first, second and third components can each be formed into a liquid, emulsion, cream, gel, paste or mousse in a conventional manner. They can also be formed into an aerosol.

The hair dye of the present invention upon usage has a pH ranging from 8 to 12, especially 8.5 to 11. At a pH less than 8, effects of the present invention are not sufficient, while at a pH exceeding 12, the resulting hair dye causes a severe scalp irritation and is therefore unsuited for practical use.

### -Examples-

### Reference Examples 1, 2 and Comparative Examples 1, 2 (two-part hair dye)

Two-part hair dyes (first component part : second component part = 1:1) as shown in Table 1 were prepared and the color shade produced by these dyes are indicated.

**Table 1**

| (wt.%) | | Ref.Example | | Comp. Ex. | |
|---|---|---|---|---|---|
| | | 1 | 2 | 1 | 2 |
| First component part | First dye [formula (3); red] | - | 0.05 | - | 0.05 |
| | First dye [formula (6); orange] | 0.1 | 0.1 | 0.1 | 0.1 |
| | First dye [formula (1); yellow] | 0.15 | - | 0.15 | - |
| | Second dye [Disperse Blue 3] | 0.1 | - | - | - |
| | Second dye [Disperse Blue 7] | - | 0.1 | - | - |
| | Blue dye A (below-described formula) | - | - | 0.1 | - |
| | Blue dye B (below-described formula) | - | - | - | 0.1 |
| | 28 wt.% Aqueous ammonia | 6 | | | |
| | Ethanol | 10 | | | |
| | Ethoxy diglycol | 10 | | | |
| | Ammonium chloride | Amount to adjust pH to 10 | | | |
| | Water | Balance | | | |
| Second component | 35 wt.% Aqueous hydrogen peroxide | 17.1 | | | |
| | Methylparaben | 0.1 | | | |
| | Phosphoric acid | Amount to adjust pH to 3.5 | | | |
| | Water | Balance | | | |
| Color shade | | Brown | Black | Orange | Red |

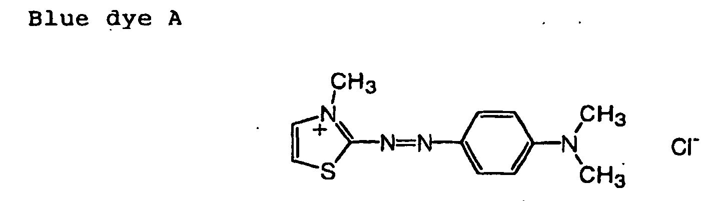

Dye prepared in Example 39 of Japanese Language Laid-Open Publication (PCT) No. Hei 8-501322

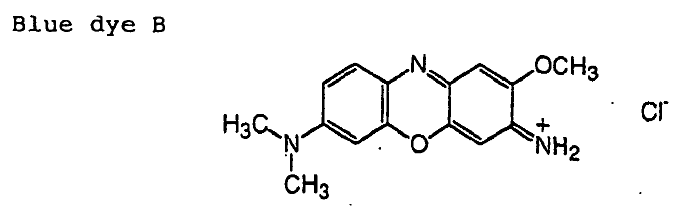

Dye prepared in Example 6 of Japanese Language Laid-Open Publication (PCT) No. Hei 8-501322

### Reference Example 3, Example 1 and Comparative Examples 3, 4 (three-part type hair dye)

Three-part type hair dyes (first component part : second component part : third component part = 1:1:0.1) as shown in Table 2 were prepared and the colors produced by them are indicated.

**Table 2**

| (wt. %) | | Ref. Ex. | Ex. | Comp. Ex. | |
|---|---|---|---|---|---|
| | | 3 | 1 | 3 | 4 |
| First component part | 28 wt.% Aqueous ammonia | 6 | | | |
| | Ethanol | 10 | | | |
| | Propylene glycol | 2 | | | |
| | Ammonium chloride | Amount to adjust pH to 10 | | | |
| | Water | Balance | | | |
| Second component part | 35 wt.% Aqueous hydrogen peroxide | 17.1 | | | |
| | Methylparaben | 0.1 | | | |
| | Phosphoric acid | Amount to adjust pH to 3.5 | | | |
| | Water | Balance | | | |
| Third Component art | First dye [formula (3); red] | - | 1 | - | 1 |
| | First dye [formula (6); orange] | 2 | 2 | 2 | 2 |
| | First dye [formula (1); yellow] | 3 | - | 3 | - |
| | second dye [Basic Blue 99] | 3 | - | - | - |
| | Second dye [Basic Blue 47] | - | 2 | - | - |
| | Blue dye A [above-described formula] | - | - | 3 | - |
| | Blue dye B [above-described formula] | - | - | - | 2 |
| | Propylene glycol | Balance | | | |
| Color shade | | Brown | Black | Orange | Red |

## Claims

1. A hair dye composition having a pH ranging from 8 to 12 and comprising a first component part containing an alkali agent and a direct dye and a second component part containing hydrogen peroxide,
wherein the first component part contains, as the direct dye; (A) a first direct dye represented by any one of the following formulas (1) to (8): 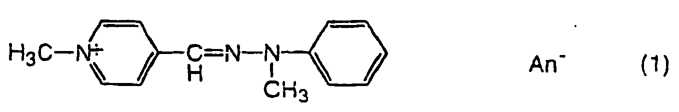 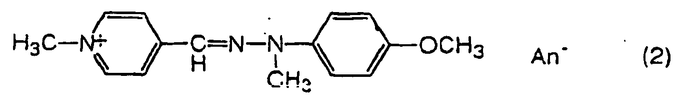 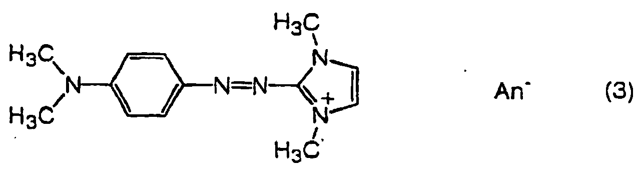 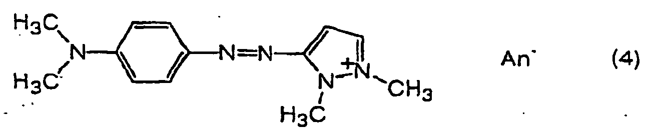 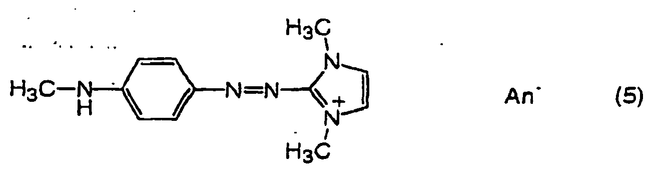 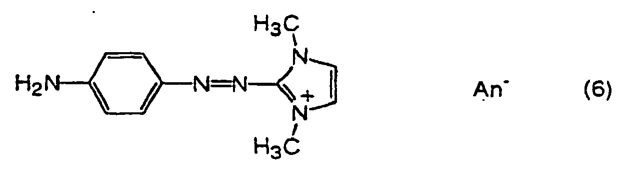 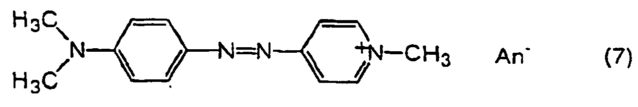 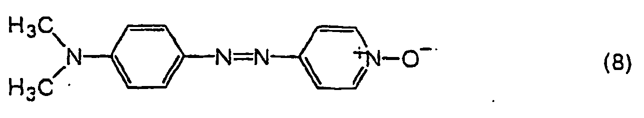 [wherein, An⁻ represents an anion]; and (B) a second direct dye selected from Basic Blue 47, Disperse Violet 1, Disperse Violet 4, Disperse Violet 15 and Disperse Violet 27.

2. A hair dye composition having a pH ranging from 8 to 12 and comprising a first component part containing an alkali agent, a second component part containing hydrogen peroxide and a third component part containing a direct dye, wherein the third component part contains, as the direct dye, (A) a first direct dye represented by any one of the following formulas (1) to (8) : 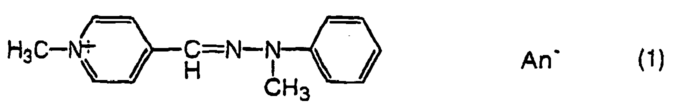 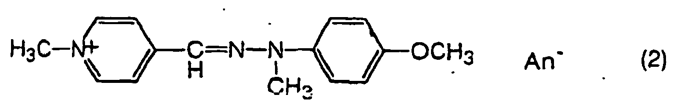 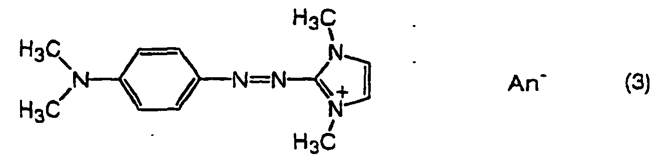 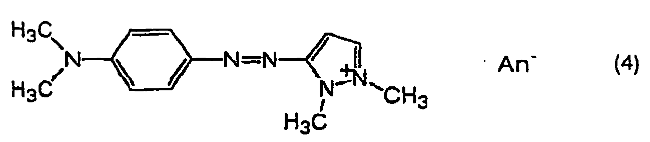 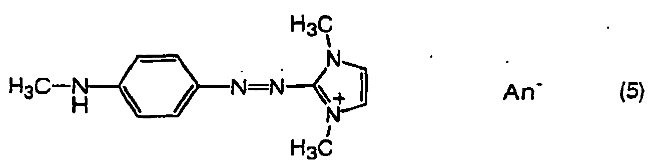 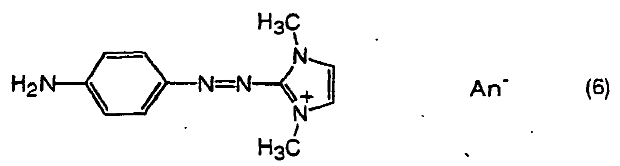 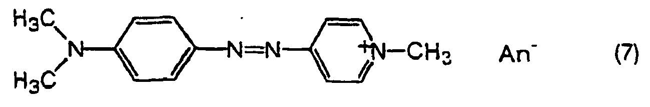 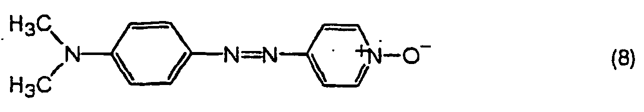 [wherein, An⁻ represents an anion]; and (B) a second direct dye selected from Basic Blue 47, Disperse Violet 1, Disperse Violet 4, Disperse Violet 15 and Disperse Violet 27.

3. A hair dyeing method, which comprises mixing a first component part and a second component part of a hair dye composition as claimed in Claim 1 or a first component, a second component and a third component of a hair dye composition as claimed in Claim 2 upon usage and then applying the resulting mixture to the hair.

## Patentansprüche

1. Haarfärbezusammensetzung mit einem pH im Bereich von 8 bis 12 und umfassend einen ersten Komponententeil, enthaltend ein Alkalimittel und einen direkt ziehenden Farbstoff, und einen zweiten Komponententeil, umfassend Wasserstoffperoxid, worin der erste Komponententeil als direkt ziehenden Farbstoff (A) einen ersten direkt ziehenden Farbstoff mit einer der folgenden Formeln (1) bis (8): 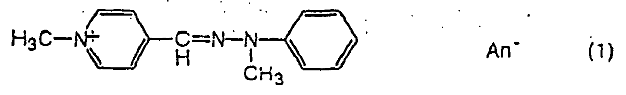 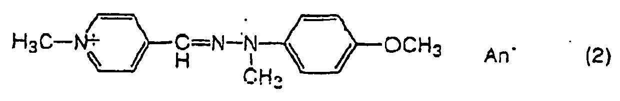 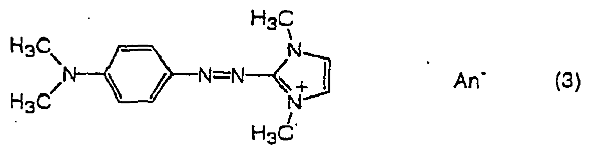 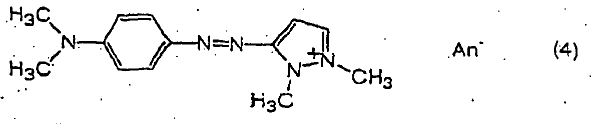 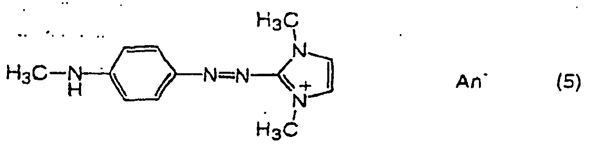 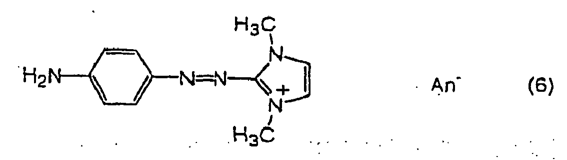 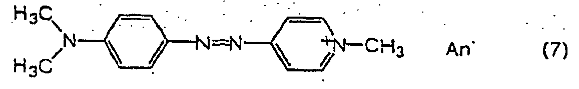 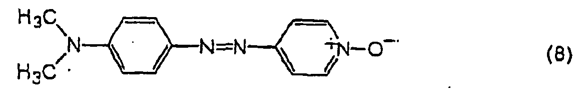
worin An⁻ ein Anion ist; und (B) einen zweiten direkt ziehenden Farbstoff enthält, ausgewählt aus Basic Blue 47, Disperse Violet 1, Disperse Violet 4, Disperse Violet 15 und Disperse Violet 27.

2. Haarfärbezusammensetzung mit einem pH von 8 bis 12 und umfassend einen ersten Komponententeil, enthaltend ein Alkalimittel, einen zweiten Komponententeil, enthaltend Wasserstoffperoxid, und einen dritten Komponententeil, umfassend einen direkt ziehenden Farbstoff, worin der dritte Komponententeil als direkt ziehenden Farbstoff (A) einen ersten direkt ziehenden Farbstoff mit einer der folgenden Formeln (1) bis (8): 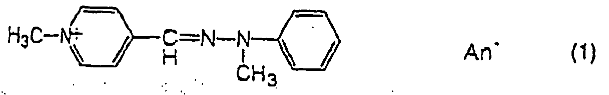 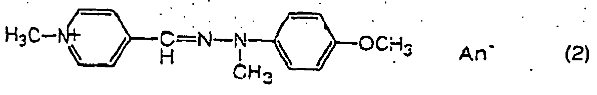 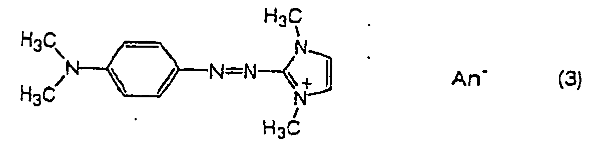 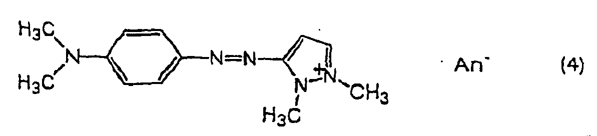 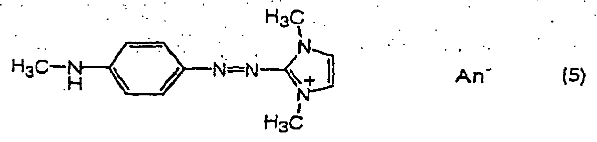 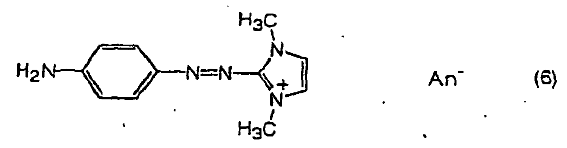 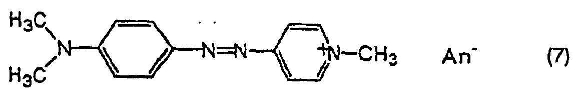 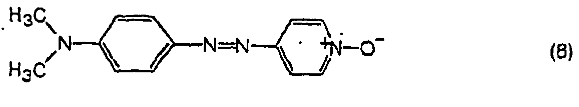
worin An⁻ ein Anion ist; und (B) einen zweiten direkt ziehenden Farbstoff enthält, ausgewählt aus Basic Blue 47, Disperse Violet 1, Disperse Violet 4, Disperse Violet 15 und Disperse Violet 27.

3. Haarfärbeverfahren, umfassend das Mischen eines ersten Komponententeils und eines zweiten Komponententeils einer Haarfärbezusammensetzung wie in Anspruch 1 beansprucht, oder einer ersten Komponente, einer zweiten Komponente und einer dritten Komponente einer Haarfärbezusammensetzung wie in Anspruch 2 beansprucht, bei der Verwendung und anschließendes Auftragen der resultierenden Mischung auf das Haar.

## Revendications

1. Composition de coloration capillaire ayant un pH se situant dans la gamme de 8 à 12 et comprenant une première partie de composants contenant un agent alcalin et un colorant direct et une seconde partie de composants contenant du peroxyde d'hydrogène,
dans laquelle la première partie de composants contient, comme colorant direct, (A) un premier colorant direct représenté par l'une quelconque des formules suivantes (1) à (8) : 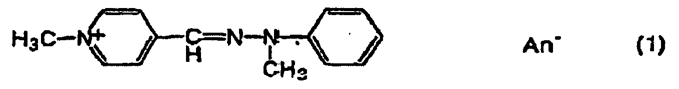 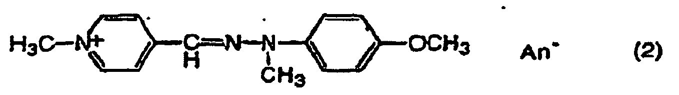 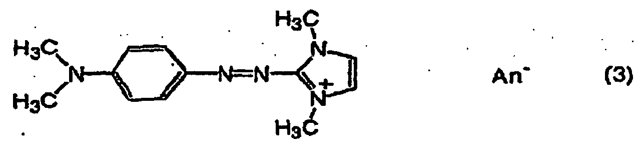 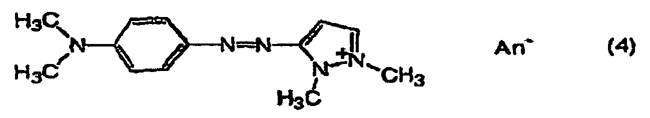 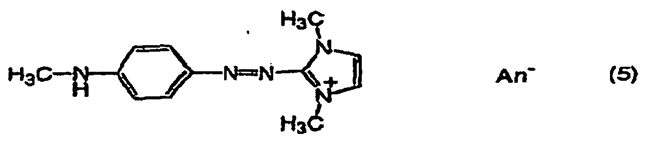 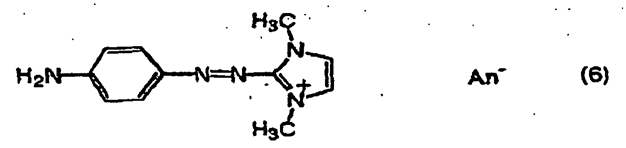 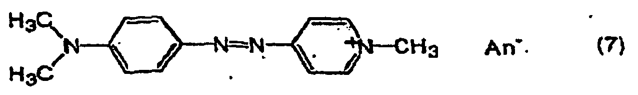 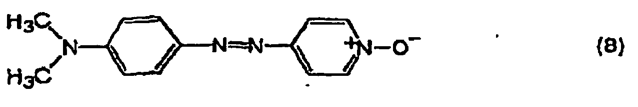 [dans lesquelles, An⁻ représente un anion] ; et (B) un second colorant direct choisi parmi le Basic Blue 47, le Disperse Violet 1, le Disperse Violet 4, le Disperse Violet 15 et le Disperse Violet 27.

2. Composition de coloration capillaire ayant un pH se situant dans la gamme de 8 à 12 et comprenant une première partie de composants contenant un agent alcalin, une seconde partie de composants contenant du peroxyde d'hydrogène et une troisième partie de composants contenant un colorant direct, dans laquelle la troisième partie de composants contient, comme colorant direct, (A) un premier colorant direct représenté par l'une quelconque des formules (1) à (8) suivantes : 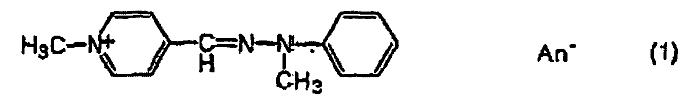 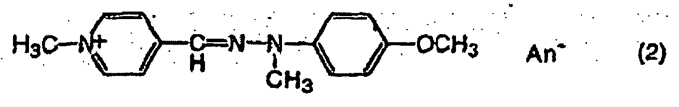 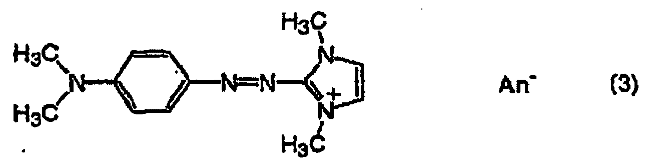 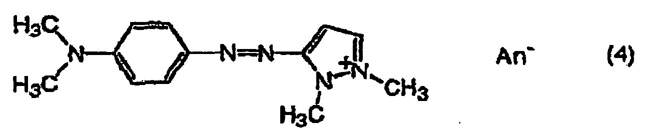 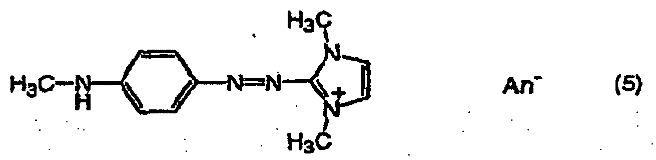 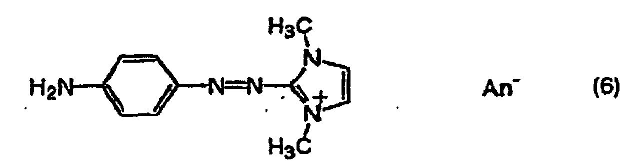 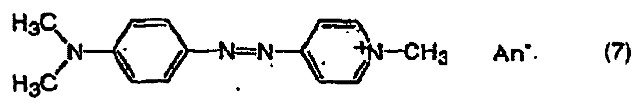 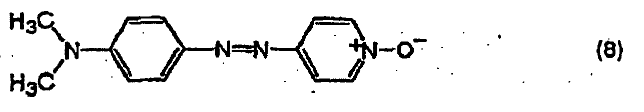 [dans lesquelles, An⁻ représente un anion] ; et (B) un second colorant direct choisi parmi le Basic Blue 47, le Disperse Violet 1, le Disperse Violet 4, le Disperse Violet 15 et le Disperse Violet 27.

3. Procédé de coloration capillaire, qui comprend le mélange d'une première partie de composants et d'une seconde partie de composants d'une composition de coloration capillaire telle que revendiquée dans la revendication 1 ou d' un premier composant, d'un second composant et d'un troisième composant d'une composition de coloration capillaire telle que revendiquée dans la revendication 2 au moment de l'utilisation et ensuite l'application du mélange résultant aux cheveux.
